(19) Europäisches Patentamt
European Patent Office
Office européen des brevets

(11) Veröffentlichungsnummer: **0 127 048**
**A2**

# (12) EUROPÄISCHE PATENTANMELDUNG

(21) Anmeldenummer: 84105437.2

(22) Anmeldetag: 14.05.84

(51) Int. Cl.³: C 07 C 69/712
A 01 N 39/02, C 07 C 121/38
C 07 C 153/09, C 07 C 149/14
C 07 F 7/08, C 07 D 231/12
//C07C143/68, C07C59/135

(30) Priorität: 27.05.83 DE 3319367

(43) Veröffentlichungstag der Anmeldung: 05.12.84 Patentblatt 84/49

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Förster, Heinz, Dr.
Am Eckbusch 47
D-5600 Wuppertal 1(DE)

(72) Erfinder: Priesnitz, Uwe, Dr
Severinstrasse 58
D-5650 Solingen 1(DE)

(72) Erfinder: Riebel, Hans-Jochem, Dr.
In der Beek 92
D-5600 Wuppertal 1(DE)

(72) Erfinder: Eue, Ludwig, Dr.
Paul-Klee-Strasse 36
D-5090 Leverkusen 1(DE)

(72) Erfinder: Schmidt, Robert R., Dr.
Im Waldwinkel 110
D-5060 Bergisch-Gladbach(DE)

## (54) Optisch aktive Diphenylether-Derivate.

(57) Neue R-Enantiomere von Diphenylether-Derivaten der Formel

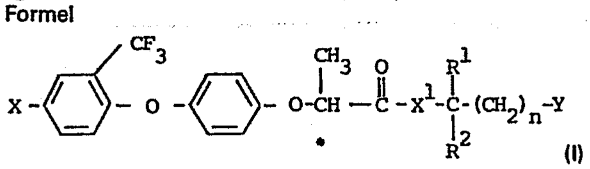

(I)

in welcher

X für Wasserstoff oder Halogen steht,

X¹ für Sauerstoff oder Schwefel steht,

R¹ und R² unabhängig voneinander für Wasserstoff oder Methyl stehen,

n für 0, 1 oder 2 steht und

Y für Trimethylsilyl, gegebenenfalls substituiertes über Stickstoff gebundenes Azolyl, Alkoxycarbonyl, Cyano oder den Rest der Formel

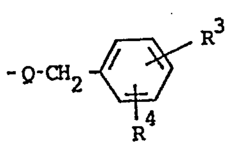

steht,

worin

Q für Sauerstoff, Schwefel, SO oder $SO_2$ steht und

R³ und R⁴ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

mehrere Verfahren zur Herstellung dieser neuen Stoffe und deren Verwendung als Herbizide.

EP 0 127 048 A2

BAYER AKTIENGESELLSCHAFT 5090 Leverkusen, Bayerwerk

Konzernverwaltung RP
Patentabteilung

Dü/Hed-c

I b

## Optisch aktive Diphenylether-Derivate

Die vorliegende Erfindung betrifft neue R-Enantiomere*) von Diphenylether-Derivaten, mehrere Verfahren zu deren Herstellung und ihre Verwendung als Herbizide.

Es ist bereits bekannt, daß zahlreiche Diphenylether-Derivate herbizide Eigenschaften besitzen (vgl. DE-OS 22 23 894). So kann zum Beispiel das Racemat des 2-/4-(2,4-dichlor-phenoxy)-phenoxy7-propionsäure-methylesters zur Bekämpfung von Unkraut eingesetzt werden. Die Wirkung dieses Stoffes ist gut, jedoch werden bei geringen Aufwandmengen einige Unkräuter nicht immer voll erfaßt.

---

*) Unter R-Enantiomeren sind im vorliegenden Fall jeweils diejenigen optisch aktiven Verbindungen zu verstehen, welche am asymmetrisch substituierten Kohlenstoffatom in der Seitenkette die R-Konfiguration aufweisen.

Es wurden nun neue R-Enantiomere von Diphenylether-Derivaten der Formel

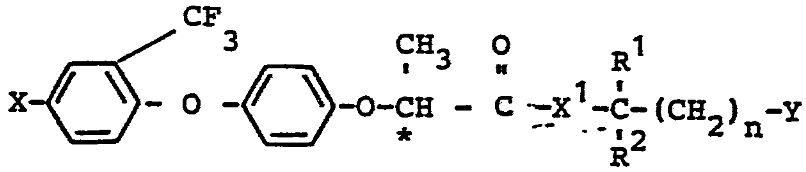

(I)

in welcher

X für Wasserstoff oder Halogen steht,

$X^1$ für Sauerstoff oder Schwefel steht,

$R^1$ und $R^2$ unabhängig voneinander für Wasserstoff oder Methyl stehen,

n für 0, 1 oder 2 steht und

Y für Trimethylsilyl, gegebenenfalls substituiertes über Stickstoff gebundenes Azolyl, Alkoxycarbonyl, Cyano oder den Rest der Formel

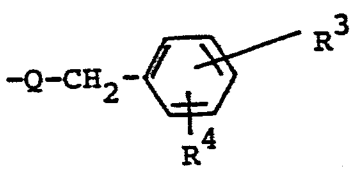

steht,

worin

Q für Sauerstoff, Schwefel, SO oder $SO_2$ steht und

$R^3$ und $R^4$ unabhängig voneinander für Wasserstoff, Halogen, Alkyl mit 1 bis 4 Kohlenstoffatomen, Alkoxy mit 1 bis

4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

gefunden.

Weiterhin wurde gefunden, daß man die neuen R-Enantiomeren von Diphenylether-Derivaten der Formel (I) erhält, wenn man

a) Phenol-Derivate der Formel

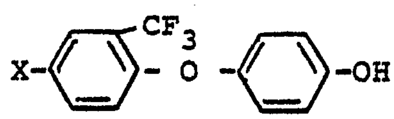

(II)

in welcher

X die oben angegebene Bedeutung hat,

mit S-Enantiomeren der Propionsäure-Derivate der Formel

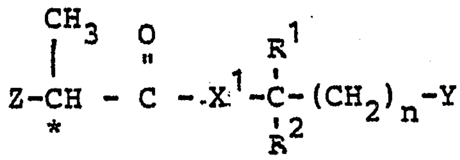

(III)

in welcher

$R^1$, $R^2$, Y, $X^1$ und n die oben angegebene Bedeutung haben und

Z für Tosylat oder Mesylat steht,

gegebenenfalls in Gegenwart eines Säureakzeptors sowie gegebenenfalls in Gegenwart eines Verdünnungsmittels umsetzt,

oder

b) R-Enantiomere von Phenoxypropionsäure-Derivaten der Formel

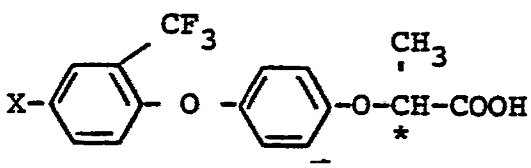

(IV)

in welcher

X die oben angegebene Bedeutung hat,

α) mit Silylchloriden der Formel

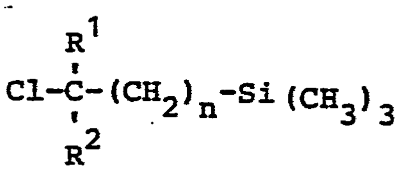

(V)

in welcher

$R^1$, $R^2$ und n die oben angegebene Bedeutung haben,

oder

ß) mit Verbindungen der Formel

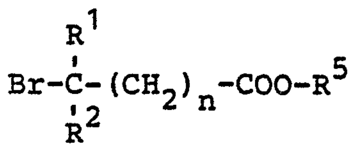

(VI)

in welcher

$R^1$, $R^2$ und n die oben angegebene Bedeutung haben und

$R^5$ für Alkyl steht,

jeweils gegebenenfalls in Gegenwart eines Säurebindemittels sowie gegebenenfalls in Gegenwart eines Verdünnungsmittels umsetzt,

oder

c) R-Enantiomere von Phenoxypropionsäurechloriden der Formel

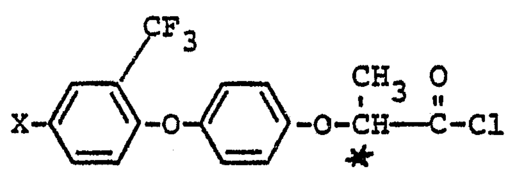

(VII)

in welcher

X die oben angegebene Bedeutung hat,

mit Verbindungen der Formel

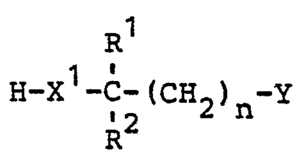

(VIII)

in welcher

$R^1$, $R^2$, $X^1$, Y und n die oben angegebene Bedeutung haben,

gegebenenfalls in Gegenwart eines Säureakzeptors sowie gegebenenfalls in Gegenwart eines Verdünnungsmittels umsetzt.

Schließlich wurde gefunden, daß sich die neuen R-Enantiomeren von Diphenylether-Derivaten der Formel (I) durch eine hervorragende herbizide Wirksamkeit auszeichnen.

Überraschenderweise besitzen die erfindungsgemäßen R-Enantiomeren der Diphenylether-Derivate der Formel (I) wesentlich bessere herbizide Eigenschaften als das aus dem Stand der Technik bekannte Racemat des 2-/4-(2,4-Dichlor-phenoxy)-phenoxy7-propionsäuremethylesters, welches ein gut wirksamer Stoff gleicher Wirkungsart ist.

Die erfindungsgemäßen R-Enantiomeren von Diphenylether-Derivaten sind durch die Formel (I) allgemein definiert.

In dieser Formel, in der das asmmetrisch substituierte Kohlenstoffatom durch ein (*) gekennzeichnet ist, steht X vorzugsweise für Wasserstoff, Fluor, Chlor oder Brom. $X^1$ steht für Sauerstoff oder Schwefel, $R^1$ und $R^2$ stehen unabhängig voneinander für Wasserstoff oder Methyl und der Index n steht für 0, 1 oder 2. Der Rest Y steht vorzugsweise für Trimethylsilyl, oder für einen über ein

Ring-Stickstoffatom gebundenen Pyrazolyl-, Imidazolyl-, 1,2,4-Triazolyl-, 1,2,3-Triazolyl- oder 1,3,4-Triazolyl--Rest, wobei jeder dieser Azolyl-Reste ein- oder mehrfach, gleichartig oder verschieden substituiert sein kann durch Fluor, Chlor, Brom, Iod, Alkyl mit 1 bis 4 Kohlenstoffatomen, Alkoxy mit 1 bis 4 Kohlenstoffatomen und/oder Phenyl. Weiterhin steht Y vorzugsweise für Alkoxycarbonyl mit 1 bis 4 Kohlenstoffatomen im Alkoxyteil, Cyano oder für den Rest der Formel

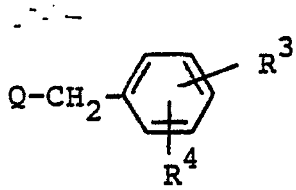

worin

Q für Sauerstoff, Schwefel, SO oder $SO_2$ steht und $R^3$ und $R^4$ unabhängig voneinander vorzugsweise für Wasserstoff, Fluor, Chlor, Brom, Iod, Alkyl mit 1 bis 4 Kohlenstoffatomen, Alkoxy mit 1 bis 4 Kohlenstoffatomen, Alkylthio mit 1 oder 2 Kohlenstoffatomen, Nitro, Cyano oder Alkoxycarbonyl mit 1 oder 2 Kohlenstoffatomen in der Alkoxygruppe stehen.

Diejenigen R-Enantiomeren der Diphenylether-Derivate der Formel (I), in denen $R^1$ für Methyl steht und $R^2$ für Wasserstoff steht, enthalten ein zweites Asymmetriezentrum in der Ester-Einheit. Die betreffenden Stoffe können daher in den folgenden Formen vorliegen:

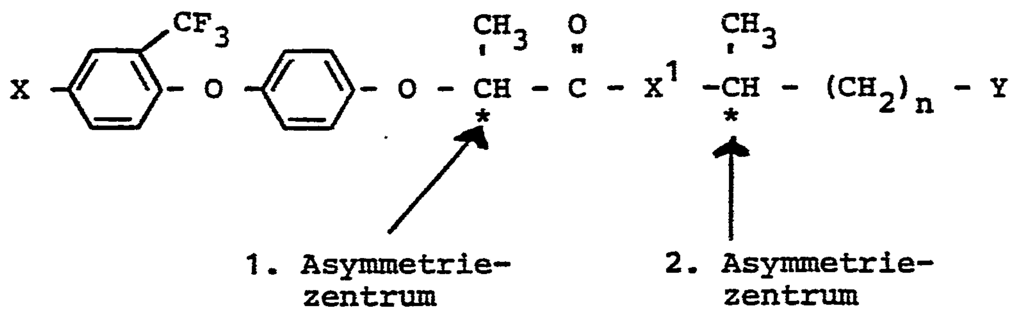


(Ia)

| Konfiguration am 1. Asymmetriezentrum | Konfiguration am 2. Asymmetriezentrum |
|---|---|
| R | R |
| R | S |
| R | R/S (Racemat) |

Die vorliegende Erfindung umfaßt sowohl diejenigen Verbindungen, die am zweiten Asymmetriezentrum die R- oder die S-Konfiguration aufweisen als auch die entsprechenden Racemate.

Verwendet man 4-(4-Chlor-2-trifluormethyl-phenoxy)-phenol und das S-Enantiomere des 2-Tosyloxy-propionsäure-(ethoxycarbonyl)-methylesters als Ausgangsstoffe, so kann der Verlauf des erfindungsgemäßen Verfahrens (a) durch das folgende Formelschema wiedergegeben werden:

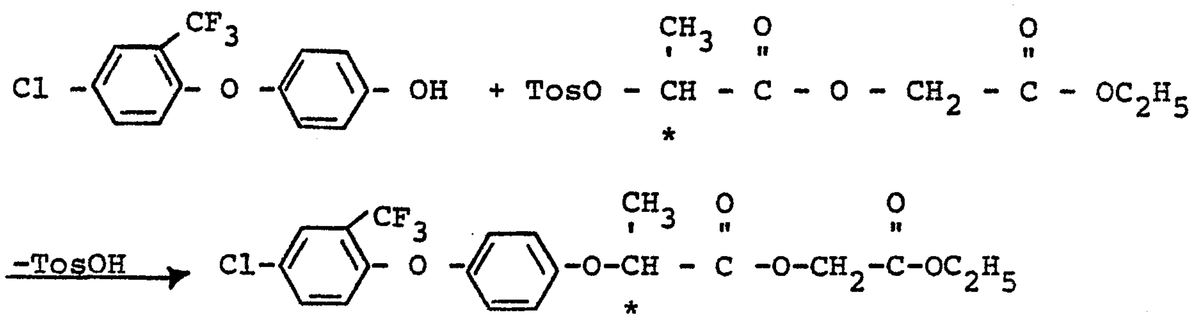


Tos = 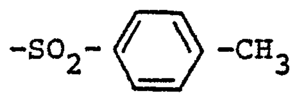 ( = Tosyl)

Verwendet man das R-Enantiomere der 2-/4-(4-Chlor-2-trifluormethyl-phenoxy)-phenoxy7-propionsäure und Chlormethyl-trimethylsilan als Ausgangsstoffe, so kann der Verlauf des erfindungsgemäßen Verfahrens (b, Variante α) durch das folgende Formelschema wiedergegeben werden:

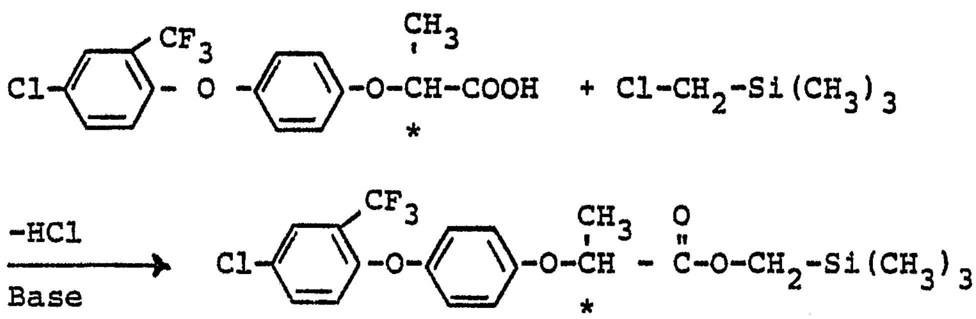


Verwendet man das R-Enantiomere der 2-/4-(4-Chlor-2-trifluormethyl-phenoxy)-phenoxy7-propionsäure und Bromessigsäureethylester als Ausgangsstoffe, so kann der Verlauf des erfindungsgemäßen Verfahrens (b, Variante ß) durch das folgende Formelschema wiedergegeben werden:

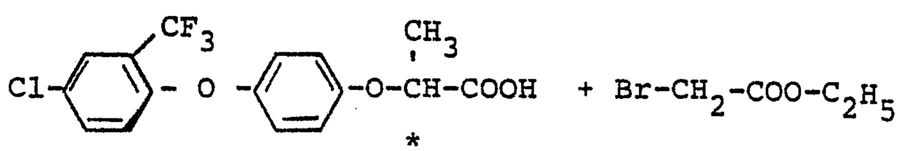

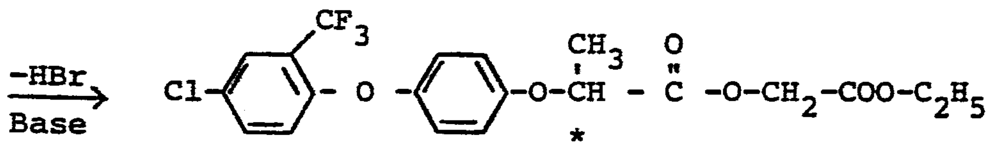


Verwendet man das R-Enantiomere des 2-[4-(4-Chlor-2-trifluormethyl-phenoxy)-phenoxy]-propionsäurechlorids und 2-(Pyrazol-1-yl)-ethanol als Ausgangsstoffe, so kann der Verlauf des erfindungsgemäßen Verfahrens (c) durch das folgende Formelschema wiedergegeben werden:

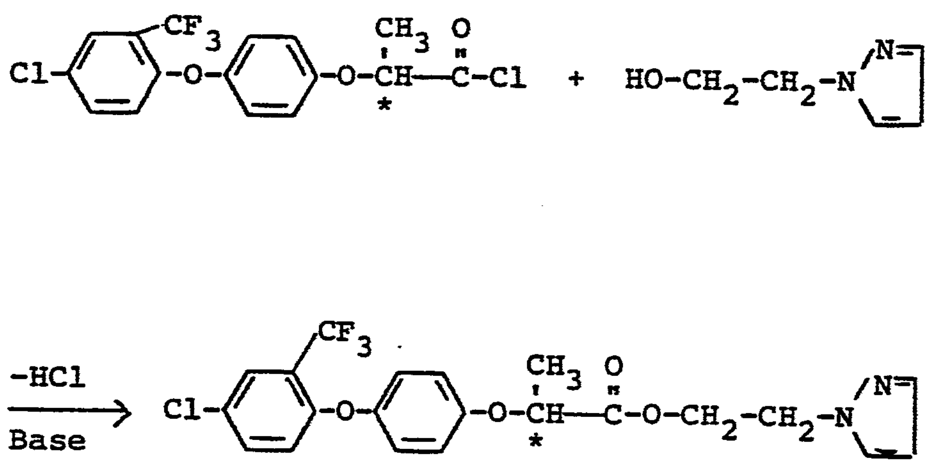


Die bei dem erfindungsgemäßen Verfahren (a) als Ausgangsstoffe benötigten Phenol-Derivate sind durch die Formel (II) definiert. In dieser Formel steht X vorzugsweise für Wasserstoff, Fluor, Chlor und Brom.

Von den Phenol-Derivaten der Formel (II) ist bisher nur das 4-[4-Chlor-2-trifluormethyl-phenoxy)-phenol bekannt (vgl. DE-OS 25 38 016). Die Stoffe der Formel

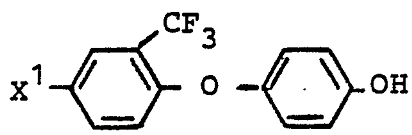

(II a)

in welcher

$X^1$ für Wasserstoff, Fluor oder Brom steht, sind neu. Sie lassen sich herstellen, indem man Trifluormethyl-benzol-Derivate der Formel

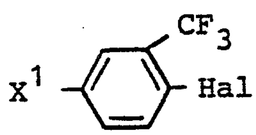

(IX)

in welcher

$X^1$ die oben angegebene Bedeutung hat und

Hal für Chlor oder Brom steht,

mit Hydrochinon in Gegenwart eines Säureakzeptors, wie zum Beispiel Calciumhydroxid, sowie in Gegenwart eines polaren Verdünnungsmittels, wie zum Beispiel Acetonitril, Dimethylformamid oder Dimethylsulfoxid, bei Temperaturen zwischen 20 und 200°C, vorzugsweise zwischen 50 und 140°C umsetzt.

Die bei dem erfindungsgemäßen Verfahren (a) weiterhin als Ausgangsstoffe benötigten S-Enantiomeren der Propionsäure-Derivate sind durch die Formel (III) definiert. In dieser Formel steht Z für Tosylat ($-O-SO_2-$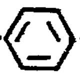$-CH_3$) oder Mesylat ($-O-SO_2-CH_3$). Der Rest $X^1$ steht für Sauerstoff oder Schwefel.

Die Reste $R^1$ und $R^2$ stehen unabhängig voneinander für Wasserstoff oder Methyl, der Index n steht für 0, 1 oder 2 und Y steht vorzugsweise für Trimethylsilyl oder für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest, wobei jeder dieser Azolyl-Reste ein- oder mehrfach, gleichartig oder verschieden substituiert sein kann durch Chlor, Fluor, Brom, Iod, Alkyl mit 1 bis 4 Kohlenstoffatomen, Alkoxy mit 1 bis 4 Kohlenstoffatomen und/oder Phenyl. Weiterhin steht Y vorzugsweise für Alkoxycarbonyl mit 1 bis 4 Kohlenstoffatomen im Alkoxyteil, Cyano oder für den Rest der Formel

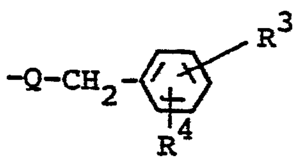

Worin Q für Sauerstoff, Schwefel, SO oder $SO_2$ steht und $R^3$ und $R^4$ unabhängig voneinander vorzugsweise für Wasserstoff, Fluor, Chlor, Brom, Iod, Alkyl mit 1 oder 2 Kohlenstoffatomen, Alkoxy mit 1 oder 2 Kohlenstoffatomen, Alkylthio mit 1 oder 2 Kohlenstoffatomen, Nitro, Cyano oder Alkoxycarbonyl mit 1 oder 2 Kohlenstoffatomen in der Alkoxygruppe.

Diejenigen Stoffe der Formel (III), in denen $R^2$ für Wasserstoff steht, enthalten im Ester-Teil ein zweites Asymmetriezentrum, das in der R- oder S-Konfiguration vorliegen kann. In den entsprechenden Racematen leistet das asymmetrisch substituierte Kohlenstoffatom im Ester--Teil keinen Beitrag zur optischen Aktivität der S-Enantiomeren der Propionsäure-Derivate der Formel (III).

Die S-Enantiomeren der Propionsäure-Derivate der Formel (III) sind bekannt oder lassen sich nach üblichen Methoden in einfacher Weise herstellen. So erhält man die S-Enantiomeren der Propionsäure-Derivate der Formel (III), indem man S-Enantiomere von Propionylchloriden der Formel

$$Z - \overset{\overset{CH_3}{|}}{\underset{*}{CH}} - CO - Cl \qquad (X)$$

in welcher

Z die oben angegebene Bedeutung hat,

mit Verbindungen der Formel

$$H-X^1-\overset{\overset{R^1}{|}}{\underset{\underset{R^2}{|}}{C}}-(CH_2)_n-Y \qquad (VIII)$$

in welcher

$X^1$, Y, $R^1$, $R^2$ und n die oben angegebene Bedeutung haben,

gegebenenfalls in Gegenwart eines Säureakzeptors sowie gegebenenfalls in Gegenwart eines Verdünnungsmittels umsetzt.

Die bei der Herstellung der S-Enantiomeren der Propionsäure-Derivate der Formel (III) als Ausgangsstoffe benötigten S-Enantiomeren von Propionylchloriden der Formel (X) sind bekannt oder lassen sich nach bekannten Methoden herstellen. Die für die Umsetzung zur Synthese der S-Enantiomeren der Propionsäure-Derivate der Formel (III) außerdem als Reaktionskomponenten benötigten Verbindungen der Formel (VIII) sind ebenfalls bekannt oder lassen sich nach bekannten Methoden in einfacher Weise herstellen.

Bei diesem Verfahren zur Herstellung der S-Enantiomeren der Propionsäure-Derivate der Formel (III) entsprechen die Umsetzungsbedingungen denen des erfindungsgemäßen Verfahrens (a) (vgl. unten).

Der Einsatz von S-Enantiomeren der Phenoxypropionsäure-Derivate der Formel (III) ist bei dem erfindungsgemäßen Verfahren (a) deshalb erforderlich, weil im Verlauf der Reaktion eine Walden'sche Umkehr am asymmetrisch substituierten Kohlenstoffatom der Propionsäure-Einheit erfolgt.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalihydroxide, Erdalkalihydroxide und -oxide, wie z.B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, 1,5-Diazabicyclo/4,3,0/non-5-en (DBN), 1,8-Diazabicyclo/5,4,0/-undec-7-en (DBU) und Pyridin.

Als Verdünnungsmittel kommen bei der Durchführung des erfindungsgemäßen Verfahrens (a) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methylisopropyl-, und Methyl-isobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei der Umsetzung nach dem erfindungsgemäßen Verfahren (a) innerhalb eines größeren Bereiches variiert werden. Im allgemeinen arbeitet man bei Temperaturen zwischen -20°C und +160°C, vorzugsweise zwischen 0°C und +140°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die Ausgangsstoffe der Formeln (II) und (III) im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren.

Die bei dem erfindungsgemäßen Verfahren (b) als Ausgangsstoffe benötigten R-Enantiomeren der Phenoxypropionsäure-Derivate sind durch die Formel (IV) definiert. In dieser Formel steht X vorzugsweise für Wasserstoff, Fluor, Chlor und Brom.

Die Verbindungen der Formel (IV) lassen sich aus den R-Enantiomeren von Estern der Formel

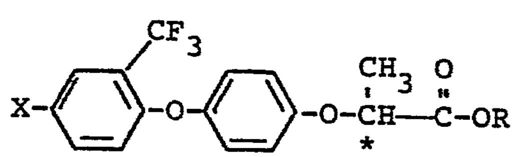

(XI)

in welcher

X die oben angegebene Bedeutung hat und

R für Alkyl, insbesondere für Alkyl mit 1 bis 4 Kohlenstoffatomen, steht,

nach üblichen Methoden durch Verseifung herstellen. Vorzugsweise verwendet man wäßrige Alkalihydroxidlaugen, wie zum Beispiel Natronlauge oder Kalilauge, als Verseifungsreagenzien.

Die Esterverseifung wird im allgemeinen in Gegenwart eines Verdünnungsmittels vorgenommen. Vorzugsweise in Betracht kommen aromatische Kohlenwasserstoffe, wie Toluol oder Xylol, ferner Alkohole, wie Methanol oder Ethanol, außerdem Ether, wie Dioxan, weiterhin Nitrile, wie Acetonitril, und auch Gemische aus organischen Solventien und Wasser.

Die Temperaturen können bei der Durchführung der Esterverseifung innerhalb eines größeren Bereiches variiert werden. Im allgemeinen arbeitet man bei Temperaturen zwischen 0°C und 160°C, vorzugsweise zwischen 20°C und 140°C.

Bei der Durchführung der Esterverseifung geht man im allgemeinen so vor, daß man den Ester der Formel (XI) in Gegenwart eines Verdünnungsmittels mit einer äquivalenten Menge oder mit einem Überschuß an Base bei der jeweils gewünschten Temperatur behandelt. Die Aufarbeitung erfolgt nach üblichen Methoden. Im allgemeinen geht man so vor, daß man das Reaktionsgemisch durch Abziehen des Lösungsmittels unter vermindertem Druck einengt, den verbleibenden Rückstand in Wasser aufnimmt, mit Mineralsäure,

wie zum Beispiel Salzsäure, ansäuert und die sich dabei abscheidende Säure der Formel (IV) abtrennt.

Die bei dem erfindungsgemäßen Verfahren (b, Variante α) weiterhin als Ausgangsstoffe benötigten Silylchloride sind durch die Formel (V) definiert. In dieser Formel haben $R^1$, $R^2$ und n diejenigen Bedeutungen, die bereits im Zusammenhang mit der Beschreibung der erfindungsgemäßen Stoffe der Formel (I) für diese Reste bzw. für den Index n genannt wurden.

Die Silylchloride der Formel (V) sind bekannt oder lassen sich in einfacher Weise nach bekannten Methoden herstellen.

Die bei dem erfindungsgemäßen Verfahren (b, Variante ß) weiterhin als Ausgangsstoffe benötigten Verbindungen sind durch die Formel (VI) definiert. In dieser Formel haben $R^1$, $R^2$ und n wiederum diejenigen Bedeutungen, die bereits im Zusammenhang mit der Beschreibung der erfindungsgemäßen Stoffe der Formel (I) für diese Reste, bzw. für den Index n genannt wurden. $R^5$ steht vorzugsweise für Alkyl mit 1 bis 4 Kohlenstoffatomen.

Diejenigen Stoffe der Formel (VI), in denen $R^1$ für Methyl und $R^2$ für Wasserstoff steht, können entweder als Racemate oder als R- bzw. S-Enantiomere vorliegen. Verwendet man bei dem erfindungsgemäßen Verfahren (b, Variante ß) die R-Enantiomeren dieser Verbindungen der Formel (VI), so entstehen diejenigen erfindungsgemäßen Stoffe, die am zweiten Asymmetriezentrum die S-Konfiguration aufweisen, weil im Zuge der Umsetzung eine Walden'sche Umkehr erfolgt. Entsprechend entstehen aus den

S-Enantiomeren der Verbindungen der Formel (VI) diejenigen erfindungsgemäßen Stoffe, die am zweiten Asymmetriezentrum die R-Konfiguration besitzen. Bei Verwendung von Racematen der Verbindungen der Formel (VI) fallen erfindungsgemäße Stoffe an, in denen das asymmetrisch substituierte Kohlenstoffatom im Ester-Teil keinen Beitrag zur optischen Aktivität der Endprodukte leistet.

Die Verbindungen der Formel (VI) sind bekannt oder lassen sich in einfacher Weise nach bekannten Methoden herstellen.

Bei der Durchführung des erfindungsgemäßen Verfahrens (b) wird sowohl in der Variante α als auch in der Variante ß vorzugsweise in Gegenwart eines Säureakzeptors und eines Verdünnungsmittels gearbeitet. Hierbei kommen als Säurebindemittel und Verdünnungsmittel vorzugsweise alle diejenigen Stoffe in Betracht, die in diesem Zusammenhang bereits im Falle des erfindungsgemäßen Verfahrens (a) als bevorzugt genannt wurden.

Die Reakionstemperaturen können auch bei dem erfindungsgemäßen Verfahren (b) sowohl in der Variante α als auch in der Variante ß innerhalb eines größeren Bereiches variiert werden. Im allgemeinen arbeitet man jeweils mit Temperaturen zwischen -20°C und +160°C, vorzugsweise zwischen 0°C und +140°C.

Das erfindungsgemäße Verfahren (b) wird sowohl in der Variante α als auch in der Variante ß im allgemeinen

unter Normaldruck durchgeführt. Es ist jedoch auch möglich, unter erhöhtem oder vermindertem Druck zu arbeiten.

Zur Durchführung des erfindungsgemäßen Verfahrens (b, Varianten $\alpha$ und ß) werden die Ausgangsstoffe der Formeln (IV) und (V) bzw. (VI) im allgemeinen in angenähert äquimolaren Mengen eingesetzt.

Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren.

Die bei dem erfindungsgemäßen Verfahren (c) als Ausgangsstoffe benötigten R-Enantiomeren von Phenoxypropionsäure-chloriden sind durch die Formel (VII) definiert. In dieser Formel steht X vorzugsweise für Wasserstoff, Fluor, Chlor oder Brom.

Die R-Enantiomeren von Phenoxypropionsäurechloriden der Formel (VII) lassen sich herstellen, indem man R-Enantiomere von Phenoxypropionsäure-Derivaten der Formel (IV) mit Chlorierungsmitteln, wie zum Beispiel Thionylchlorid, gegebenenfalls unter Verwendung eines Katalysators, wie zum Beispiel Dimethylformamid, gegebenenfalls in Gegenwart eines Verdünnungsmittels,

wie zum Beispiel 1,2-Dichlorethan oder Methylenchlorid, bei Temperaturen zwischen 10°C und 100°C umsetzt. Die Aufarbeitung erfolgt nach üblichen Methoden. Im allgemeinen geht man so vor, daß man die flüchtigen Komponenten unter vermindertem Druck abdestilliert.

Die bei dem erfindungsgemäßen Verfahren (c) weiterhin als Ausgangsstoffe benötigten Verbindungen sind durch die Formel (VIII) definiert. In dieser Formel haben $R^1$, $R^2$, $X^1$, Y und n vorzugsweise diejenigen Bedeutungen, die bereits im Zusammenhang mit der Beschreibung der erfindungsgemäßen Verbindungen der Formel (I) für diese Reste bzw. für diesen Index als bevorzugt genannt wurden.

Die Verbindungen der Formel (VIII) sind bekannt oder lassen sich nach bekannten Methoden in einfacher Weise herstellen.

Bei der Durchführung des erfindungsgemäßen Verfahrens (c) wird vorzugsweise in Gegenwart eines Säureakzeptors und eines Verdünnungsmittels gearbeitet. Hierbei kommen als Säurebindemittel und Verdünnungsmittel vorzugsweise alle diejenigen Stoffe in Betracht, die in diesem Zusammenhang bereits im Falle des erfindungsgemäßen Verfahrens (a) als bevorzugt genannt wurden.

Die Reaktionstemperaturen können auch im Falle des erfindungsgemäßen Verfahrens (c) innerhalb eines größeren Bereiches variiert werden. Im allgemeinen arbeitet man bei Temperaturen zwischen -20°C und +160°C, vorzugsweise zwischen 0°C und +140°C.

Das erfindungsgemäße Verfahren (c) wird im allgemeinen unter Normaldurck durchgeführt. Es ist jedoch auch möglich, unter erhöhtem oder vermindertem Druck zu arbeiten.

Zur Durchführung des erfindungsgemäßen Verfahrens (c) werden die Ausgangsstoffe der Formeln (VII) und (VIII) im allgemeinen in angenähert äquimolaren Mengen eingesetzt. Es ist jedoch auch möglich, eine der beiden Komponenten in einem größeren Überschuß zu verwenden. Die Umsetzungen werden im allgemeinen in einem geeigneten Verdünnungsmittel und in Gegenwart eines Säureakzeptors durchgeführt, und das Reaktionsgemisch wird mehrere Stunden bei der jeweils erforderlichen Temperatur gerührt. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

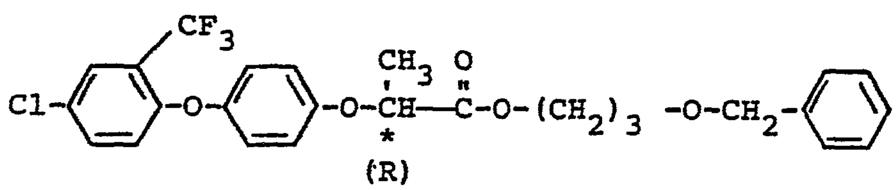

(I-1)

Ein Gemisch aus 14,4 g (0,05 Mol) 4-(4-Chlor-2-trifluormethyl-phenoxy)-phenol, 8 g (0,06 Mol) Kaliumcarbonat und 19,7 g (0,05 Mol) des S-Enantiomeren des 2-Tosyloxy-propionsäure-3-(benzyloxy)-n-propylesters in 100 ml Acetonitril wurde 12 Stunden unter Rückfluß erhitzt. Nach dem Abkühlen auf Raumtemperatur wurde das Reaktionsgemisch in 250 ml Wasser gegossen. Das entstehende Gemisch wurde zweimal mit je 100 ml Toluol extrahiert, und die vereinigten organischen Phasen wurden durch Abziehen des Lösungsmittels eingeengt. Der verbleibende Rückstand wurde durch kurzes Erhitzen im Hochvakuum von Resten an flüchtigen Anteilen befreit. Man erhielt auf diese Weise 14 g (55 % der Theorie) an dem R-Enantiomeren des 2-/4-(4-Chlor-2-trifluormethyl-phenoxy)-phenoxy7-propionsäure-3-(benzyloxy)-n-propylesters.

Brechungsindex: $n_D^{20}$ = 1,5576

Drehwert: $/\alpha 7_D^{24}$ = + 3,5°
(1-molare Lösung in Chloroform; Küvettenlänge 10 cm).

Nach der im Beispiel 1 angegebenen Methode oder auch nach anderen erfindungsgemäßen Verfahren wurden die in den folgenden Beispielen aufgeführten Stoffe hergestellt. Die Drehwerte wurden dabei jeweils an einer 1-molaren Lösung in Chloroform gemessen. Die Küvettenlänge betrug jeweils 10 cm.

Beispiele 2

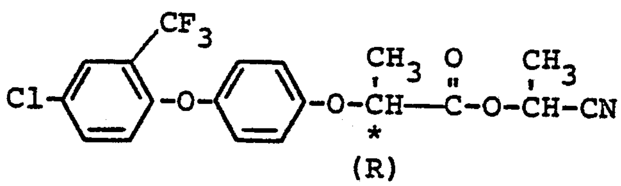

(I-2)

Drehwert: $[\alpha]_D^{24}$ = + 8,6°
Brechungsindex: $n_D^{20}$ = 1,5183

Beispiel 3

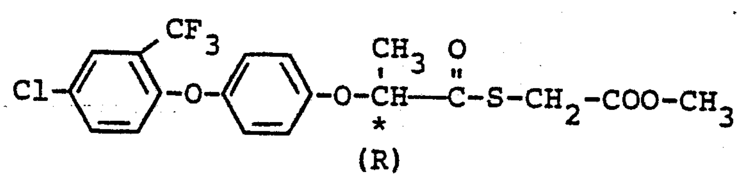

(I-3)

Brechungsindex: $n_D^{20}$ = 1,5430
Drehwert: $[\alpha]_D^{24}$ = + 3,0°

Beispiel 4

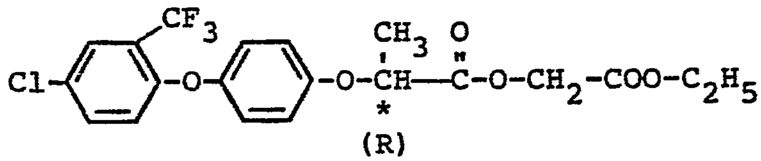

(I-4)

Drehwert: $[\alpha]_D^{24}$ = + 10,9°

Beispiel 5

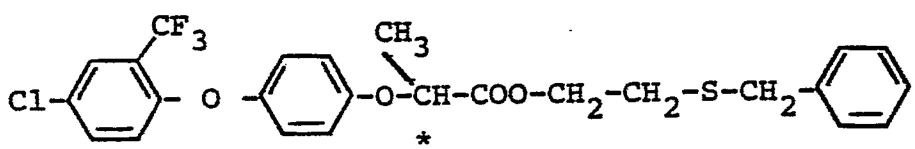

(I-5)

Ausbeute: 55 % der Theorie
Brechungsindex $n_D^{20}$: 1,5576
Drehwert: $[\alpha]_D^{24}$ = + 3,5°

Beispiel 6

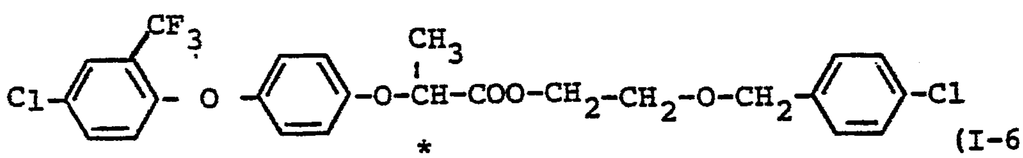

(I-6)

Ausbeute: 53 % der Theorie
Drehwert: $[\alpha]_D^{24}$ = + 2,3°
Brechungsindex: $n_D^{20}$ = 1,5412

Beispiel 7

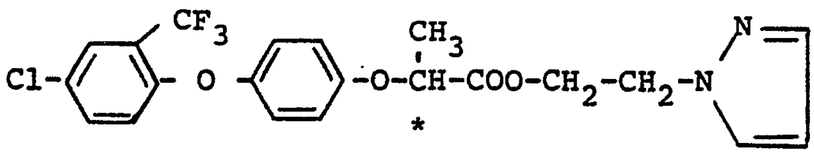

(I-7)

Ausbeute: 25 % der Theorie
Drehwert: $[\alpha]_D^{24} = + 3,2°$

Beispiel 8

Herstellung des Ausgangsproduktes der Formel:

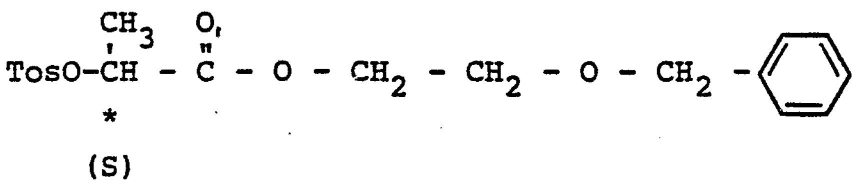

(III-1)

5,25 g (0,02 Mol) des S-Enantiomeren des Milchsäurechloridtosylates wurden bei 20°C unter Rühren in ein Gemisch aus 3,32 g (0,02 Mol) Glykol-mono-benzyl-ether, 2 g (0,02 Mol) Triethylamin und 50 ml Toluol gegeben. Man rührte weitere 14 Stunden bei 70°C und arbeitete dann auf, indem man das Reaktionsgemisch mit 100 ml Wasser versetzte, dann mehrfach mit Toluol extrahierte, die vereinigten organischen Phasen trocknete und durch Abziehen des Lösungsmittels unter vermindertem Druck einengte. Man erhielt auf diese Weise 6,3 g (80,5 % der Theorie) an S-Enantiomerem des

2-Tosyloxy-propionsäure-2-(benzyloxy)-ethylesters.

Drehwert: $[\alpha]_D^{24}$= -10,2°

Nach der in dem Beispiel 8 angegebenen Methode wurden auch die in der folgenden Tabelle formelmäßig aufgeführten Ausgangsstoffe der Formel (III) hergestellt.

Tabelle 1

$$Z-\underset{*\,(S)}{\overset{CH_3}{CH}}-CO-X^1-\underset{R^2}{\overset{R^1}{C}}-(CH_2)_n-Y \qquad (III)$$

| Bsp.Nr. | Verbindung | Z | $-X^1-\underset{R^2}{\overset{R^1}{C}}-(CH_2)_n-Y$ | Drehwert $[\alpha]_D^{24}$ |
|---|---|---|---|---|
| 9 | (III - 2) | TosO | 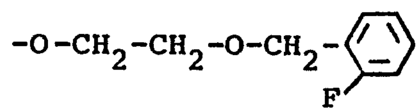 | - 9,7° |
| 10 | (III - 3) | TosO | 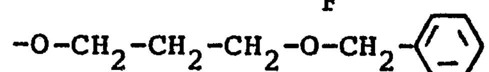 | - 10,3° |
| 11 | (III - 4) | TosO | 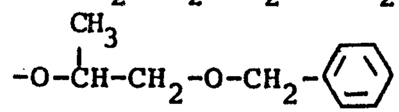 | - 4,1° |
| 12 | (III - 5) | TosO | 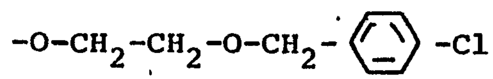 | - 7,6° |
| 13 | (III - 6) | TosO | 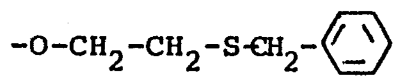 | - 10,9° |
| 14 | (III - 7) | TosO | 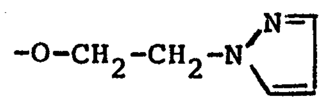 | - 11,4° |
| 15 | (III - 8) | TosO | 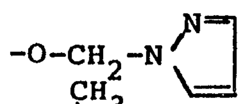 | - 13,7° |
| 16 | (III - 9 ) | TosO | $-O-\underset{*\,(S)}{\overset{CH_3}{CH}}-COO-C_2H_5$ | - 17,8° |

Tabelle 1 (Fortsetzung)

| Bsp.Nr. | Verbindung | Z | $-X^1-\overset{R^1}{\underset{R^2}{C}}-(CH_2)_n-Y$ | Drehwert $[\alpha]_D^{24}$ |
|---|---|---|---|---|
| 17 | (III-10) | TosO | $-O-CH_2-COO-C_2H_5$ | - 11,6° |
| 18 | (III-11) | TosO | $-O-CH_2-Si(CH_3)_3$ | - 11,4° |

Beispiel 19

Herstellung des Ausgangsproduktes der Formel

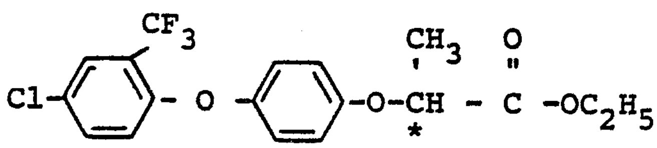

(XI-1)

Ein Gemisch aus 14,4 g (0,05 Mol) 4-(4-Chlor-2-trifluormethyl-phenoxy)-phenol, 13,6 g (0,05 Mol) des S-Enantiomeren des 2-Tosyloxy-propionsäureethylesters und 8 g (0,06 Mol) Kaliumcarbonat in 100 ml Acetonitril wurde 3 Stunden unter Rückfluß erhitzt. Danach wurde aufgearbeitet, indem man das Reaktionsgemisch absaugte, das Filtrat einengte, den verbleibenden Rückstand in Methylenchlorid löste, die entstehende Lösung einmal mit Wasser wusch und nach dem Trocknen unter vermindertem Druck eingengte. Der verbleibende Rückstand wurde durch leichtes Erwärmen im Hochvakuum von Resten an flüchtigen Anteilen befreit. Man erhielt auf diese Weise 18,8 g (96,9 % der Theorie) an R-Enantiomerem des 2-(4-(4-Chlor-2-trifluormethylphenoxy)-phenoxy)-propionsäure-ethylesters.

Drehwert: $[\alpha]_D^{24}$ = + 6,2°

Beispiel 20

Herstellung des Ausgangsproduktes der Formel:

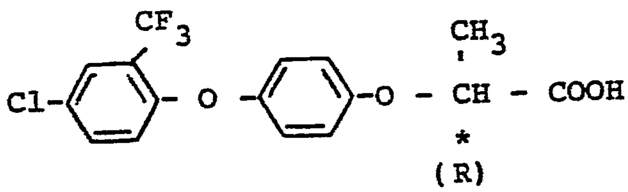

(IV-1)

Zu einer intensiv gerührten Mischung von 38,8 g (0,01 Mol) des R-Entantiomeren des 2-[4-(4-Chlor-2-trifluormethylphenoxy)-phenoxy]-propionsäureethylesters in 150 ml Xylol wurden bei 110°C 8 g (0,2 Mol) Natriumhydroxid in 50 ml Wasser getropft. Man rührte 15 Minuten bei 110°C nach, kühlte ab und versetzte das Reaktionsgemisch mit 200 ml Wasser.

Die wäßrige Phase wurde abgetrennt, mit konzentrierter, wäßriger Salzsäure angesäuert und zweimal mit je 300 ml Ether extrahiert. Die vereinigten Etherextrakte wurden über Natriumsulfat getrocknet und dann eingeengt. Man erhielt 22 g (61 % der Theorie) an R-Enantiomerem der 2-[4-(4-Chlor-2-trifluormethylphenoxy)-phenoxy]-propionsäure.

Drehwert: $[\alpha]_D^{20} = + 3,6°$

Beispiel 21

Herstellung des Ausgangsproduktes der Formel:

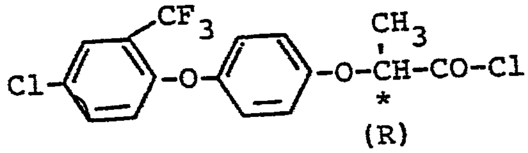

(VII-1)

In eine Mischung aus 252,4 g (0,7 Mol) des R-Enantiomeren der 2-/4-(4-Chlor-2-trifluormethyl-phenoxy)-phenoxy7-propionsäure, 500 ml Methylenchlorid und 0,5 ml Dimethylformamid wurden bei 20°C unter Rühren 100 g (0,84 Mol) Thionylchlorid eingetropft. Danach wurde weitere 4 Stunden bei 40°C gerührt und dann aufgearbeitet, indem man das Lösungsmittel und überschüssiges Thionylchlorid unter vermindertem Druck abzog. Der verbleibende Rückstand wurde durch kurzes Erwärmen im Hochvakuum von Resten an flüchtigen Anteilen befreit. Man erhielt auf diese Weise 262 g (98,8 % der Theorie) an R-Enantiomerem des 2-/4-(4-Chlor-2-trifluormethyl-phenoxy)-phenoxy7-propionsäurechlorids in Form eines Öles.
Brechungsindex: $n_D^{20}$ = 1,5371

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe (I-1), (I-2) und (I-4) zeigen in diesem Test eine sehr gute herbizide Wirksamkeit bei der selektiven Bekämpfung von Sorghum, Setaria, Digitaria und Alopecurus in Rüben und Sojabohnen.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe (I-3) und (I-4) zeigen in diesem Test eine sehr gute herbizide Wirksamkeit bei der selektiven Bekämpfung von Sorghum und Echinochloa in Rüben.

Patentansprüche

1) R-Enantiomere von Diphenylether-Derivaten der Formel

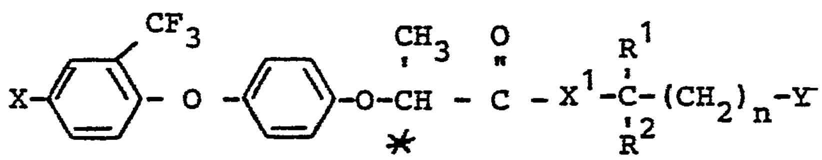

(I)

in welcher

X für Wasserstoff oder Halogen steht,

$X^1$ für Sauerstoff oder Schwefel steht,

$R^1$ und $R^2$ unabhängig voneinander für Wasserstoff oder Methyl stehen,

n für 0, 1 oder 2 steht, und

Y für Trimethylsilyl, gegebenenfalls substituiertes über Stickstoff gebundenes Azolyl, Alkoxycarbonyl, Cyano oder den Rest der Formel

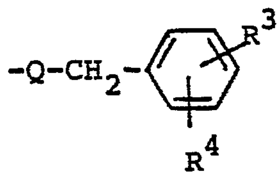

steht,

worin

Q für Sauerstoff, Schwefel, SO oder $SO_2$ steht und

$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen.

2) R-Enantiomere von Diphenylether-Derivaten der Formel (I), in denen

X für Wasserstoff, Fluor, Chlor oder Brom steht,

$X^1$ für Sauerstoff oder Schwefel steht,

$R^1$ und $R^2$ unabhängig voneinander für Wasserstoff oder Methyl stehen,

n für 0, 1 oder 2 steht und

Y für Trimethylsilyl oder für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein- oder mehrfach, gleichartig oder verschieden substituiert sein kann durch Fluor, Chlor, Brom, Iod, Alkyl mit 1 bis 4 Kohlenstoffatomen, Alkoxy mit 1 bis 4 Kohlenstoffatomen und/oder Phenyl,

und

Y außerdem für Alkoxycarbonyl mit 1 bis 4 Kohlenstoffatomen im Alkoxyteil, Cyano oder für den Rest der Formel

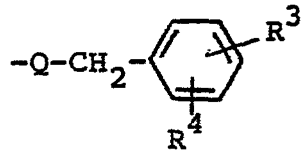 steht,

worin

Q für Sauerstoff, Schwefel, SO oder $SO_2$ steht und

$R^3$ und $R^4$ unabhängig voneinander für Wasserstoff, Fluor, Chlor, Brom, Iod, Alkyl mit 1 oder 2 Kohlenstoffatomen, Alkylthio mit 1 oder 2 Kohlenstoffatomen, Alkoxy mit 1 oder 2 Kohlenstoffatomen, Nitro, Cyano oder Alkoxycarbonyl mit 1 oder 2 Kohlenstoffatomen in der Alkoxygruppe stehen.

3) Verfahren zur Herstellung von R-Enantiomeren von Diphenylether-Derivaten der Formel

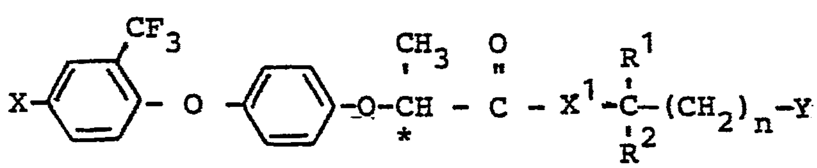 (I)

in welcher

X für Wasserstoff oder Halogen steht,

$X^1$ für Sauerstoff oder Schwefel steht,

$R^1$ und $R^2$ unabhängig voneinander für Wasserstoff oder Methyl stehen,

n für 0, 1 oder 2 steht und

Y für Trimethylsilyl, gegebenenfalls substituiertes über Stickstoff gebundenes Azolyl, Alkoxycarbonyl, Cyano oder den Rest der Formel

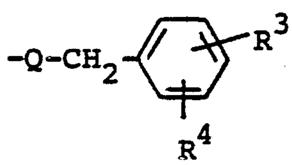 steht,

worin

Q für Sauerstoff, Schwefel, SO oder $SO_2$ steht und

$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

dadurch gekennzeichnet, daß man

a) Phenol-Derivate der Formel

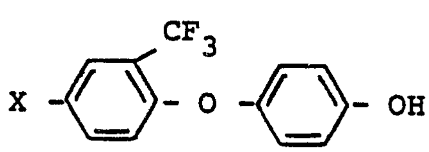 (II)

in welcher

X die oben angegebene Bedeutung hat,

mit S-Enantiomeren der Propionsäure-Derivate der Formel

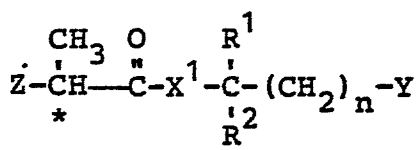

(III)

in welcher

$R^1$, $R^2$, Y und $X^1$ und n die oben genannte Bedeutung haben und
Z für Tosylat oder Mesylat steht,

gegebenenfalls in Gegenwart eines Säureakzeptors sowie gegebenenfalls in Gegenwart eines Verdünnungsmittels umsetzt, oder

b) R-Enantiomere von Phenoxypropionsäure-Derivaten der Formel

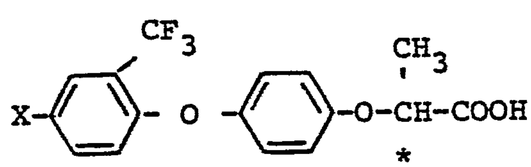

(IV)

in welcher

X die oben angegebene Bedeutung hat,

α) mit Silylchloriden der Formel

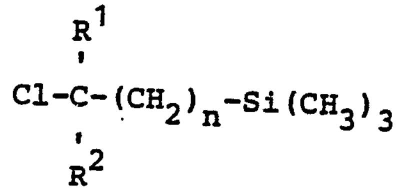

(V)

in welcher

$R^1$, $R^2$ und n die oben angegebene Bedeutung haben, oder

β) mit Verbindungen der Formel

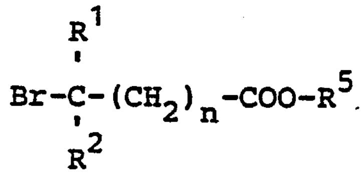

(VI)

in welcher

$R^1$, $R^2$ und n die oben angegebene Bedeutung haben und

$R^5$ für Alkyl steht,

jeweils gegebenenfalls in Gegenwart eines Säuremittels sowie gegebenenfalls in Gegenwart eines Verdünnungsmittels umsetzt, oder

c) R-Enantiomere von Phenoxypropionsäurechloriden der Formel

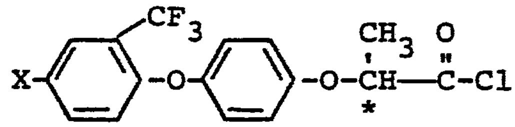

(VII)

in welcher

X die oben angegebene Bedeutung hat,

mit Verbindungen der Formel

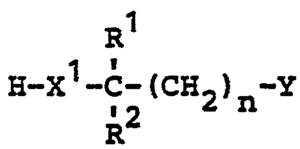

(VIII)

in welcher

$R^1$, $R^2$, $X^1$, Y und n die oben angegebene Bedeutung haben,

gegebenenfalls in Gegenwart eines Säureakzeptors sowie gegebenenfalls in Gegenwart eines Verdünnungsmittels umsetzt.

4) Herbizide Mittel, gekennzeichnet durch einen Gehalt an mindestens einem R-Enantiomeren eines Diphenylether-Derivates der Formel (I).

5) Verwendung von R-Enantiomeren von Diphenylether-Derivaten der Formel (I) als Herbizide.

6) Verfahren zur Bekämpfung von Unkräutern, dadurch gekennzeichnet, daß man R-Enantiomere von Diphenylether-Derivaten der Formel (I) auf die Unkräuter und/oder deren Lebensraum ausbringt.

7) Verfahren zur Herstellung von herbiziden Mitteln, dadurch gekennzeichnet, daß man R-Enantiomere von Diphenylether-Derivaten der Formel (I) mit Streckmitteln und/oder oberflächenakiven Stoffen vermischt.

8) R-Enantiomer des Diphenylether-Derivates der Formel

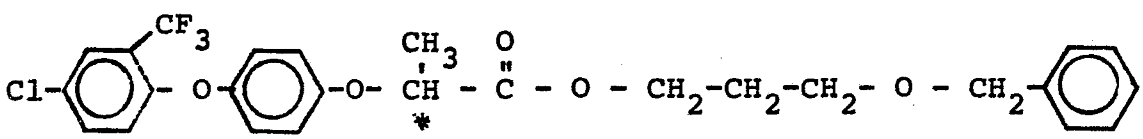

9) R-Enantiomer des Diphenylether-Derivates der Formel

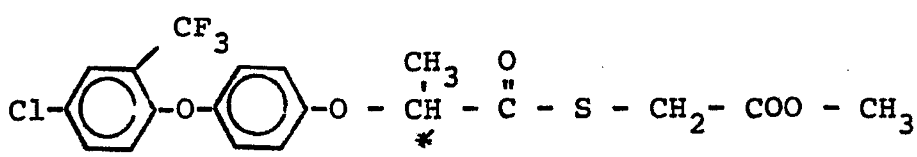

1o) R-Enantiomer des Diphenylether-Derivates der Formel